# EUROPEAN PATENT APPLICATION

(11) **EP 4 424 254 A1**
(43) Date of publication of application: **04.09.2024**
(21) Application number: 24150556.9
(22) Date of filing: 05.01.2024
(51) Int. Cl.: A61B 17/86

(54) **ORTHOPEDIC BONE FASTENERS**

(30) Priority: 28.02.2023 US 202318175582
(71) Applicant: Globus Medical, Inc, Audubon, PA 19403 (US)
(72) Inventor: GLERUM, Chad, Pennsburg, PA 18073 (US); VENT, Justin, Havertown, PA 19083 (US); PALMER, Rory, Spring City, PA 19475 (US); JAMES, Luke, Wilmington, DE 19810 (US); HANSELL, Noah, King of Prussia, PA 19406 (US); DEVANNEY, Brandyn, Sellersville, PA 18960 (US); YACOUB, George, Lansdale, PA 19446 (US); ALLEN, Caelan, Ambler, PA 19002 (US)
(74) Representative: Morabito, Sara

(57) **Abstract**

Orthopedic bone fasteners, assemblies, and methods for spinal and trauma applications. The bone fastener may include a screw head and a shaft with an external thread helically wound around the shaft. Each thread section includes a crest, a root, and leading and following flanks connecting the crest to adjacent roots. The external thread may have a hook and latch style thread geometry. The leading and/or following flanks may define a hook, such as an undercut, configured to improve bone purchase, resist axial and lateral forces, improve pull-out strength, minimize micromotion, and/or lag bone fragments together.

## Description

### FIELD OF THE INVENTION

The present application relates generally to orthopedic fixation devices, such as bone fasteners, for example, for spine surgery or trauma applications.

### BACKGROUND OF THE INVENTION

Many types of spinal irregularities cause pain, limit range of motion, or injure the nervous system within the spinal column. These irregularities may result from, without limitations, trauma, tumor, disc degeneration, and disease. Often, these irregularities are treated by immobilizing a portion of the spine. This treatment typically involves affixing a bone screw to one or more vertebrae and connecting the bone screws to an elongate spinal rod to form a spinal construct that stabilizes the spine. Traditional bone screws may allow for micromotion, for example, when axial and lateral motions are applied to the screw shank. As such, there remains a need for bone screws designed to decrease motion of the screw within the bone as well as resist axial and lateral forces applied to the screw shank.

In the case of a trauma, one or more bone screws may be used to lag bone fragments together for healing purposes. Traditional screw thread profiles may help to prevent fragments from pulling apart, but the fragments may still be prone to lateral separation. As such, there remains a need for bone screws designed such that the bone fragments can better resist micromotion and can remain lagged together for faster and more effective healing.

### SUMMARY OF THE INVENTION

To meet this and other needs, orthopedic implants, assemblies, and methods are provided. The bone fasteners may be configured for spinal and/or trauma applications. The external thread of the fastener may include leading and/or following flanks defining a hook, such as an undercut. In the case of spinal applications, the hook thread profile may be configured to help improve bone purchase, resist axial and lateral forces, and/or improve pull-out strength. The bone fastener may be implanted, for example, in open, semi-open, or percutaneous approaches to the spine with or without the assistance of navigation and/or a robotic system. In the case of trauma applications, the hook thread profile may be configured to lag bone fragments together, prevent lateral separation, and/or minimize micromotion.

According to one embodiment, an orthopedic bone fastener includes a screw head and a shaft extending along a central longitudinal axis between a proximal end and a distal end. The screw head defines a drive recess and the shaft is configured for engaging bone. An external thread is helically wound around the shaft. Each thread section has a crest, a root, and leading and following flanks connecting the crest to adjacent roots. The leading flank faces toward the distal end and is configured to enter into bone first and the following flank faces toward the proximal end of the bone fastener. At least one of the leading and following flanks comprises an undercut, thereby forming a hooked thread profile.

The bone fastener may include one or more of the following features. The undercut may be a concave recessed surface with a radius of curvature extending between the crest and the root. The undercut may be a radial undercut. The hooked thread profile may extend along an entire length of the shaft. The distal end of the bone fastener may include a relief cut configured to preserve bone upon entry of the bone fastener. The crest may be convexly curved. The external thread may include a single lead, dual lead, or multi-lead thread. The external thread may include a dual lead thread with a dual radial undercut.

According to one embodiment, an orthopedic bone fastener configured for lagging bone fragments together may include a screw head and a shaft extending along a central longitudinal axis between a proximal end and a distal end. A dual lead thread is helically wound around the shaft including a first thread section and a second thread section. Each thread section has a crest, a root, and leading and following flanks connecting the crest to adjacent roots. The leading flank faces toward the distal end and is configured to enter into bone first and the following flank faces toward the proximal end of the bone fastener. Each of the first and second thread sections comprise an undercut, thereby forming a hook-like thread configured to help resist axial and lateral motions. Preferably, the hook-like thread is configured to help resist axial and lateral motions of the screw.

The bone fastener may include one or more of the following features. The dual lead thread may have a repeating pattern of dual radial undercuts and V-shaped threads. The first and second thread sections may comprise dual radial undercuts facing toward one another. An area between the first thread section and the second thread section may form a U-shaped groove such that the crests of adjacent thread sections point toward one another. An area between the second thread section and the next first thread section may form a V-shaped groove such that the crests of adjacent thread section point away from one another. The first thread section may include an angled leading flank and a first undercut on the following flank. The second thread section may include a second undercut on the leading flank and an angled following flank.

According to another embodiment, a method for lagging bone fragments together may include one or more of the following steps in any suitable order: (1) drilling a hole through the bone fragments; (2) determining screw length with a depth gauge; (3) tapping the bone to facilitate screw insertion; and (4) inserting the bone fastener having a hooked thread profile into the opening by applying a torque to compress the fracture fragments together. The hooked thread profile may help to hook the bone fragments together to securely retain the bone reduction, resist lateral separation, resist micromotion, and/or provide a more stable environment for healing.

According to another embodiment, an orthopedic bone fastener configured to increase pullout strength includes a screw head and a shaft having a distal tip extending along a central longitudinal axis. The shaft has a proximal portion and a distal portion. An external thread is helically wound around the shaft. Preferably, the external thread includes a first thread section and a second thread section. Each thread section has a crest, a root, and leading and following flanks connecting the crest to adjacent roots. The leading flank faces toward the distal tip and is configured to enter into bone first and the following flank faces toward the screw head of the bone fastener. The following flank comprises a hook configured to resist axial and lateral forces. Preferably, the hook is configured to resist axial and lateral forces on the screw.

The bone fastener may include one or more of the following features. The external thread may include non-linear leading and following flanks. The hook may include a circular recess. The hook may define a radial undercut with a concave recessed surface extending between the crest and the root. The leading flank may comprise a convex curved profile. The distal portion of the shaft may include a single lead thread configured to engage cancellous bone and the proximal portion of the shaft may include a double lead thread configured to engage cortical bone.

According to another embodiment, a method for securing a bone fastener in a vertebra may include one or more of the following steps in any suitable order: (1) accessing the spine (e.g., a posterior aspect of the spine), for example, in a minimally invasive manner; (2) inserting the bone fastener having a hooked thread profile into the vertebra (e.g., like a pedicle screw); (3) attaching a modular tulip head to the head of the bone fastener; and (4) positioning a rod into the modular tulip head and attaching a locking cap to secure the rod to the bone fastener. The hooked thread profile of the bone fastener may help to improve bone purchase, resist axial and lateral forces, and/or improve pull-out strength.

According to another embodiment, a manufacturing method for forming the hooktype thread having one or more undercuts may include one or more of the following steps in any suitable order: (1) providing a lathe or other suitable machine and a plurality tool bits or single point cutting tools; (2) positioning a roughing tool against the shaft of the bone fastener to cut a thread minor of the root and/or optionally one or more crest angles for the crest; (3) using a back radius form tool to cut the back root radius of a first undercut; (4) using a front radius form tool to cut the front root radius of a second undercut; (5) optionally, replacing the back and front radius form tools with a dual radius form tool to simultaneously cut the front and back root radii of the undercuts; and (6) following a custom tool path for each tool bit including adjusting pitch/feed rate at different points along the tool path.

According to another embodiment, a bone fastener assembly includes an inner core, an outer sleeve, and a cap for securing the sleeve to the core. The core may include an elongated rod and enlarged head. The outer sleeve is received over the rod of the core and includes one or more threads configured to engage bone. The end cap permanently couples the sleeve to the core. Components of the bone fastener assembly may be made partly or fully from carbon fiber or an equivalent radiolucent material to minimize visual artifacts during magnetic resonance imaging (MRI) and computerized tomography (CT) scans, minimize scattering during radiation therapy, offer superior fatigue strength, and/or provide modularity for more efficient manufacturing.

Also provided are kits including implants of varying types and sizes, for example, having varying thread forms, various instruments and tools including tool bits for machining the thread forms, and other components for performing the procedures.

Preferably, at least one of the embodiments described in the present disclosure may comprise any one of the features of the other embodiments disclosed therein.

### BRIEF DESCRIPTION OF THE DRAWINGS

A more complete understanding of the present invention, and the attendant advantages and features thereof, will be more readily understood by reference to the following detailed description when considered in conjunction with the accompanying drawings, wherein:
FIGS. 1A-1B show cross-sectional views of a bone fastener including a radial undercut on the thread form which acts as a retention mechanism according to one embodiment;
FIGS. 2A-2B show cross-sectional views of a bone screw with a dual radial undercut according to one embodiment;
FIG. 3 is a close-up perspective view of the distal tip of the bone screw of FIG. 2A including a relief cut according to one embodiment;
FIG. 4 is a close-up view of the screw thread including a hook profile with a curved leading flank and an inner cup with a radial undercut according to one embodiment;
FIGS. 5A-5B show perspective and cross-sectional views, respectively, of a bone fastener with the hooked thread profile of FIG. 4 including a single-to-dual lead thread according to one embodiment;
FIGS. 6A-6B show perspective and cross-sectional views, respectively, of a bone screw with a triangular thread profile without undercuts;
FIGS. 7A-7B show perspective and cross-sectional views, respectively, of a bone fastener with a screw thread having radial undercuts and truncated crests according to one embodiment;
FIG. 8 shows a close-up view of the screw thread of FIGS. 7A-7B including the radial undercuts and truncated crests according to one embodiment;
FIGS. 9A-9D show a series of custom tool tips configured to form the crest angles and/or undercuts to achieve a desired thread form, for example, as shown in FIG. 8;
FIG. 10 shows an example of a custom tool path for manufacturing a desired thread form, for example, as shown in FIG. 8;
FIGS. 11A-11B show perspective and cross-sectional views, respectively, of a bone fastener having a single to dual lead thread according to one embodiment;
FIGS. 12A-12B show perspective and cross-sectional views, respectively, of a bone fastener having a dual to quad lead thread according to one embodiment;
FIGS. 13A-13B show perspective and cross-sectional views, respectively, of a bone fastener having a buttress thread that propagates as a single thread and transitions into a dual lead thread according to one embodiment;
FIGS. 14A-14B show cross-sectional views of examples of buttress thread profiles with different pitches and thread depths;
FIGS. 15A-15B show cross-sectional views of examples of buttress thread profiles with different pitches, thread depths, and curved roots;
FIGS. 16A-16B show cross-sectional views of examples of thread profiles with radial undercuts and different pitches and thread depths;
FIGS. 17A-17B show cross-sectional views of examples of thread profiles with radial undercuts and different pitches;
FIGS. 18A-18C show a bone fastener assembly having a titanium core and carbon fiber sleeve according to one embodiment; and
FIG. 19 is a bone fastener assembly having a carbon fiber core and a titanium sleeve according to another embodiment.

### DETAILED DESCRIPTION OF THE INVENTION

Embodiments of the disclosure are generally directed to orthopedic implants, assemblies, and methods. The bone fasteners may be configured for spinal and/or trauma applications. Specifically, some embodiments are directed to bone fasteners configured to secure one or more spinal rods. The bone fastener may form part of a modular bone fastener assembly. The bone fastener may include a modular screw configured to be inserted into bone with or without navigation and/or robotic assistance. After screw installation, a modular head may be deployed and attached to the modular screw. These implants may be used in open and percutaneous approaches to the spine. Although described with reference to the spine, it will be appreciated that the devices described herein may be applied to other orthopedic locations in the body and other medical procedures, such as trauma applications. For example, the bone fasteners may also be configured to lag together bone fragments.

Additional aspects, advantages and/or other features of example embodiments of the invention will become apparent in view of the following detailed description. It should be apparent to those skilled in the art that the described embodiments provided herein are merely exemplary and illustrative and not limiting. Numerous embodiments or modifications thereof are contemplated as falling within the scope of this disclosure and equivalents thereto.

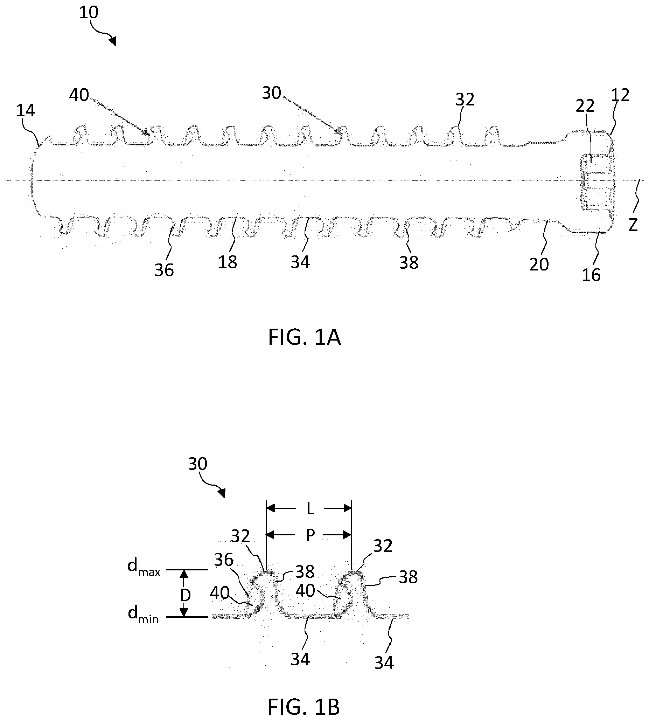

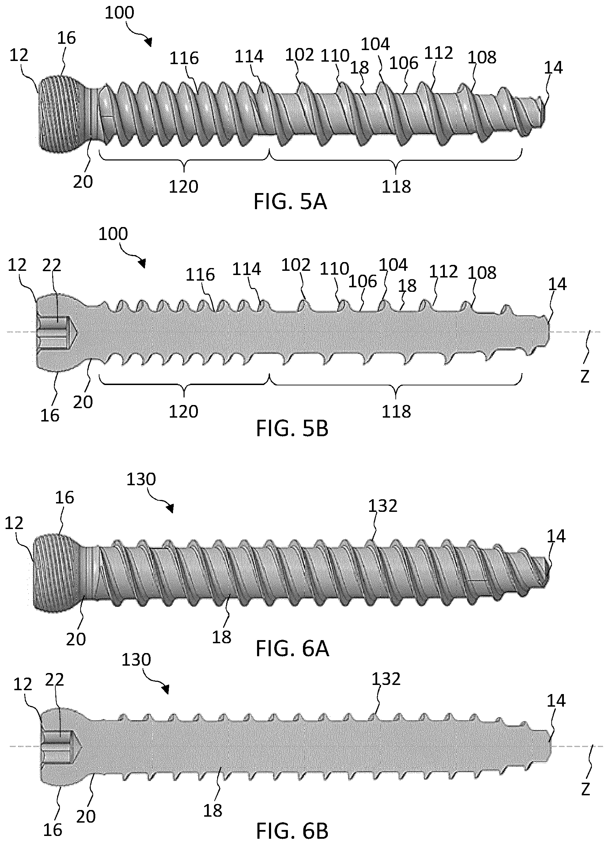

Referring now to FIGS. 1A-1B, an orthopedic fixation device, implant, or bone fastener 10 is shown according to one embodiment. The implant or bone fastener 10 may include a bone screw, anchor, clamp, or the like configured to engage bone. In the embodiment shown, the bone fastener 10 is a bone screw, which extends from a proximal end 12 to a distal end 14 along a central longitudinal axis Z. The proximal end 12 may include an enlarged head 16. The screw head 16 may be partially or fully rounded, cylindrical, spherical, or otherwise configured to engage bone and/or interface with a modular tulip element or head (e.g., for securing a spinal rod). The screw head 16 may be smooth, threaded (as shown in FIG. 5A), or provided with a roughened or textured surface. Examples of bone fasteners and other implants and rod constructs are described in more detail, for example, in U.S. Patent No. 10,368,917, which is incorporated by reference herein in its entirety for all purposes. The bone screw 10 may also be configured to lag bone fragments together for healing purposes.

The screw head 16 may define one or more drive and/or engagement surfaces 22, for example, that can be engaged by a screw-driving instrument or other instrument. In one embodiment, the bone screw head 16 defines a hexalobular socket or drive recess 22 for driving the screw 10 into bone. It will be appreciated that any suitably shaped tool engagement drive recess 22 may be provided. The bone fastener 10 includes an elongated shank or shaft 18 connected to the screw head 16 by a neck portion 20. The neck portion 20 may be reduced in diameter relative to the head 16 and/or the same or greater in diameter relative to the shaft 18. The shaft 18 may be generally cylindrical, conical, or tapered, for example. The shaft 18 terminates at a distal tip at the distal end 14, which may be blunt, pointed, or otherwise configured to engage bone.

With further emphasis of the close-up view shown in FIG. 1B, the shaft 18 of the screw 10 includes one or more threads 30. The screw thread 30 may include an external thread with a helical ridge defined by a helical groove wrapped around the shaft 18. The thread 30 may extend the entire length or a partial distance along the length of the shaft 18. Each screw thread section 30 has a crest 32 at the top-most point or outer-most surface of the ridge and a root 34 at the bottom-most point or inner-most surface of the groove. The flanks 36, 38 are the sides that connect the crest 32 and adjacent roots 34. The leading flank 36, which forms one side of the thread 30, connects the root 34 to the crest 32. The leading flank 36 is configured to enter into the bone or opening first. In other words, the leading flank 36 faces the distal end 14 of the screw 10. The following flank 38, which forms the other side of the thread 30, connects the crest 32 to the next root 34. The following flank 38 faces the proximal end 12 of the screw 10.

The profile of the screw threads 30 may be defined using one or more parameters. The thread major or major diameter dₘₐₓ is the diameter of an imaginary cylinder (concentric to the central screw axis Z) that envelopes and touches the crests 32 of the external threads 360. The thread minor, root diameter, or minor diameter dₘᵢₙ is the diameter of an imaginary cylinder (concentric to the central axis Z) that touches the thread roots 34. Although described as an imaginary cylinder (e.g., a straight thread), it will be appreciated that the surface may also be an imaginary cone in the case of a tapered thread. A distance between the crest 32 and the root 34 is a height or depth D of the thread 30. The pitch P is an axial distance between equivalent points on adjacent threads (e.g., a distance between adjacent crests 32). The lead L is the linear distance moved by the shaft 18 in one revolution (360°) of the shaft 18. The thread 30 may include a single lead, dual lead, triple lead, or multi-lead start. In the embodiment shown in FIG. 1B, the thread 30 includes a single lead L, and the lead L equals the pitch P.

One or both of the flanks 36, 38 may include an undercut 40. The undercut 40 may be on either one or two sides of the fastener in a single or multiple lead helical profile. The undercut 40 may include a concaved surface including a curvature, bevel, or an area recessed inward and thereby forming the flank 36, 38 or a portion thereof. In one embodiment, the flank 36, 38 defines a concave curvature such as a radial undercut. For example, the radial undercut 40 may by defined in a portion of the leading flank 36. The radial undercut 40 may cover the entire leading flank 36 or a portion thereof. For example, the undercut 40 may extend from the root 34 and up a distance toward the crest 32. The following flank 38 may have a straight, concave, or convex profile.

In the embodiment shown in FIGS. 1A-1B, the leading flank 36 defines a partial radial undercut 40 and the following flank 38 generally has a straight profile. It will be appreciated that these flanks 36, 38 may be reversed or otherwise configured. The crest 32 may be slightly curved or rounded with a convex face pointing toward the distal end 14 of the screw 10. In this embodiment, all of the crests 32 point in the same direction. The root 34 may be generally flattened or planar between the thread sections 30, but it will be appreciated that the root 34 may also be curved, tapered, or contoured.

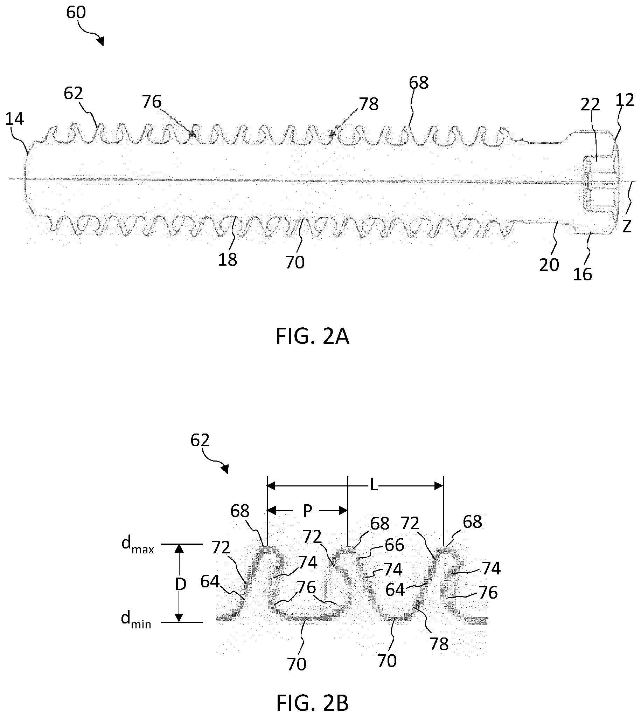

Turning now to FIGS. 2A-2B, an orthopedic fixation device, implant, or bone fastener 60 is shown according to another embodiment. Similar to bone screw 10 and labeled with like elements, bone screw 60 includes enlarged head 16 and threaded shank 18 configured to engage bone. In this embodiment, the single lead thread 30 of screw 10 is replaced by a dual lead thread 62 with dual radial undercuts 76.

As best seen in FIG. 2B, the dual lead thread 62 includes a first thread section 64 and a second thread section 66, which repeat in an alternating fashion as the helixes wind around the shaft 18. Each thread 62, 64 includes a crest 68, root 70, leading flank 72, and following flank 74. In this embodiment, the first thread section 64 includes an angled leading flank 72 and an undercut 76 on the following flank 74. The second thread section 66 includes an undercut 76 on the leading flank 72 and an angled following flank 74. The first and second thread sections 64, 66 may include dual radial undercuts 76 facing toward one another. An area between the first thread section 64 and the second thread section 66 may form a U-shaped groove such that the crests 68 of adjacent thread sections 64, 66 point toward one another. An area between the second thread section 66 and the next first thread 64 section may form a V-shaped groove such that the crests 68 of adjacent thread sections 64, 66 point away from one another. In this manner, the dual lead thread 62 may provide a screw thread geometry with a repeating pattern of dual radial undercuts 76 and V-shaped threads 78, thereby forming a hook-like thread configured to help resist axial and lateral motions and micromotion.

In the embodiment shown in FIG. 2B, the thread 62 includes a dual lead L, and the lead L is greater than the pitch P, for example, double the pitch P or greater. The area between the first thread section 64 and the second thread section 66 may form a generally U-shaped configuration such that the crests 68 of the adjacent thread sections 64, 66 point toward one another. The undercuts 76, similar to undercut 40, may include a radial undercut. The dual radial undercuts 76 may be facing one another between respective thread sections 64, 66. The crests 32 may be slightly curved or rounded with a convex face pointing toward one another. The root 34 may be generally flattened or planar between the thread sections 64, 66 or may be slightly curved or concave to connect the adjacent undercuts 76.

The area between the second thread section 66 and the next first thread section 64 may form a generally V-shaped groove 78. The following flank 74 of the second thread section 66 may have an inclined surface or plane extending from the crest 68 to the root 70. The leading flank 72 of the next first thread section 64 may have an inclined surface or plane extending from the root 70 to the next crest 68. In this manner, the following flank 74 of the second thread section 66 and the leading flank 72 of the next first thread section 64 may slope toward one another and meet at the root 70. The root 70 may be concavely curved to connect the angled flanks 72, 74 or may be otherwise configured.

In the case of lagging bone fragments together, the undercuts 40, 76 on the thread form may act as a retention mechanism for the bone fragments to resist lateral separation. The thread profile is configured to create a hook and latch style thread such that fragments can better resist micromotion and can remain lagged together for faster healing. The advantage of these types of threads 30, 62 with undercuts 40, 76 is the bone fragments may be hooked together to help resist pulling apart under normal loading conditions, which may more securely retain bone reduction and provide a more stable environment for healing.

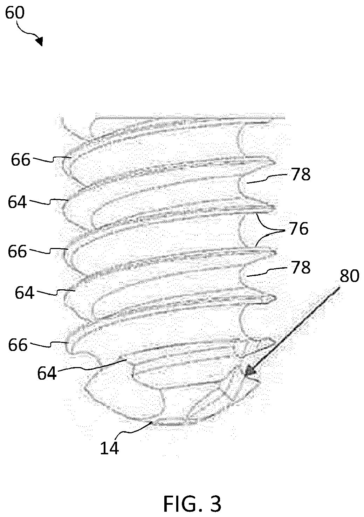

Turning now to FIG. 3, the distal end 14 of the screw 10, 60 may include a lead in or relief cut 80. The relief cut 80 at the distal end 14 of the fastener may help to allow the undercut profile to preserve the bone upon entry of the fastener 10, 60. Without a proper lead in, bone may be removed preventing the undercut 40, 76 from engaging around the bone. The lead in 80 may include a truncated version of the thread 30, 64, 66 with a ramp-type geometry creating compression of bone before the hook portion begins. This lead in or relief cut 80 may help to preserve and strengthen the interface between the thread and bone. Although FIG. 3 depicts a dual lead embodiment, it will be appreciated that a similar lead in or relief cut may be applied to a single lead or other multi-lead screw thread.

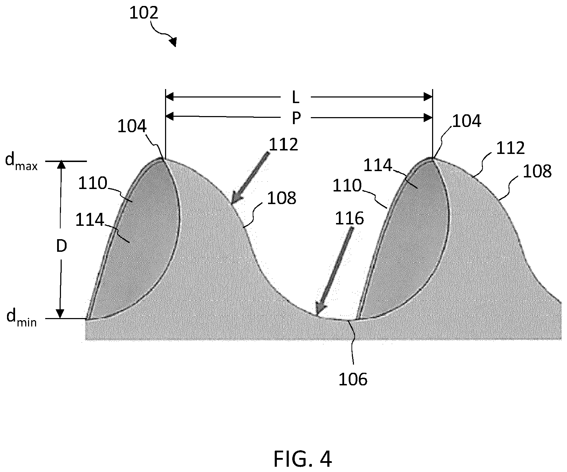

Turning now to FIGS. 4 and 5A-5B, an orthopedic fixation device, implant, or bone fastener 100 is shown according to another embodiment. Similar to bone screws 10 and 60 and labeled with like elements, bone screw 100 includes enlarged head 16 and threaded shank 18 configured to engage bone. In this embodiment, the thread 102 has a hook-like profile with a curved flank profile 112 to create the back of a hook and circular inner cup 116 to create an inner portion of the hook. The screw 100 may also include a single lead thread 118, which transitions to a dual lead thread 120 optimized for both cortical and cancellous bone.

The thread geometry may utilize a non-linear profile and circular geometries to create a hook-like shape which may relieve stresses within the bone as well as resist axial and lateral motions applied to the screw shank 18. The hooked screw may help to decrease motion of the screw 100 within bone to allow for faster boney re-growth and stronger spinal constructs particularly in osteoporotic bone. The thread shape is configured to allow for less bone to be disturbed during insertion and to maximize the pull-out strength of the screw while preventing lateral motions.

With further emphasis of the close-up view shown in FIG. 4, the shaft 18 of the screw 100 includes one or more threads 102. The screw thread 102 may include an external thread with a helical ridge defined by a helical groove wrapped around the shaft 18. Each screw thread section 102 has a crest 104, a root 106, and flanks 108, 110 that connect the crest 104 and adjacent roots 106. In this embodiment, the thread 102 defines a hook-like feature such that a curved flank profile 112 creates one side of the hook and the opposite side defines an undercut or inner recess 114 which creates a cup 116 and inner portion of the hook.

One or both of the flanks 108, 110 may include the undercut 114. The undercut 114 may include a concave surface including a curvature, bevel, or an area recessed inward and thereby forming the flank 108, 110. In the embodiment shown, the leading and following flanks 108, 110 may be reversed in type when compared to screw 10. In other words, the leading flank 108, configured to enter into the bone or opening first, may define the curved flank profile 112. The curved profile 112 may be generally convex and rounded, for example, with a generally circular or spherical curvature. The following flank 110 may define the undercut or inner recess 114. Similar to undercut 40, the undercut 114 may be generally circular, spherical, radial, or the like.

The threads 102 may run consistently throughout the length of the screw 100 or may vary to create a thread pattern optimized for both cortical and cancellous bone. In particular, the thread 30 may include a single lead, dual lead, triple lead, or multi-lead start. In the embodiment shown in FIG. 4, the thread 102 includes a portion with a single lead L, and the lead L equals the pitch P. As shown in FIG. 5A-5B, the single lead thread 118 may transition to a double lead thread 120. The distal portion of the screw shank 18 may include the single lead 118 and the proximal portion of the screw shank 18 may include the double lead 120. The double lead thread 120 has a lead L greater than the pitch P, for example, double the pitch P of single lead 118. The single lead portion 118 may be configured to optimize purchase in cancellous bone and the double lead portion 120 may be configured to optimize purchase in cortical bone of a vertebra. The thread profile may be adjusted for screw diameters, for example, ranging from 4.0mm to 10.5mm, but the geometry is such that the shape is configured to help resist axial and lateral screw motions in all sizes.

In the case of screws used with spinal constructs (e.g., attached to one or more spinal rods), the hook-like shape of the thread 102 may help to relieve stresses within the bone as well as resist axial and lateral motions applied to the screw shank 18. The hooked screw may help to decrease motion of the screw 100 within bone to allow for faster boney re-growth and stronger spinal constructs. The hooked non-standard thread geometry may allow for the screw 100 to undergo more lateral forces and provide for an increase in pull-out strength when a force is applied opposite to the insertion direction. This allows posterior fixation construct integrity to be maintained, improved bone purchase, and better boney growth.

The embodiment shown in FIGS. 5A-5B with the hook thread profile may be contrasted with FIGS. 6A-6B, which show a bone screw 130 with a generally triangular thread profile. The triangular thread form may form a V-thread, for example, based on an isosceles triangle or buttress threads based on a scalene triangle. FIGS. 6A-6B show a helical thread 132 that is not hooked in any way and does not contain any undercuts. The straight profile of bone screw 130 may not provide for the same advantages as the hooked profile. In particular, the rounded features of the threads 102 for bone screw 100 and the subsequent hook profile results in an increased area for bone to reside between the screws inner and outer profile while reducing stress points on the bone so that it maintains its structural integrity when axial or lateral forces are applied compared to screw 130 with the triangular thread profile. The hooked thread geometry may undergo more lateral forces without displacing from its initial position and without loosening under higher cyclical loads. This allows posterior fixation construct integrity to be maintained and for better boney growth in osteoporotic bone. It also allows for an increase in pull-out strength due to the larger undisturbed boney pockets left behind after screw insertion and an improvement in the geometry of these boney pockets to maximize the strength of what is left behind.

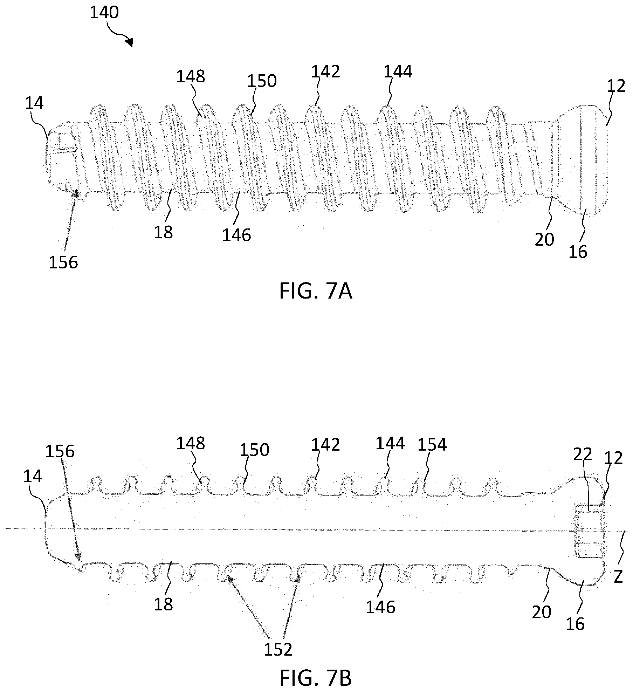

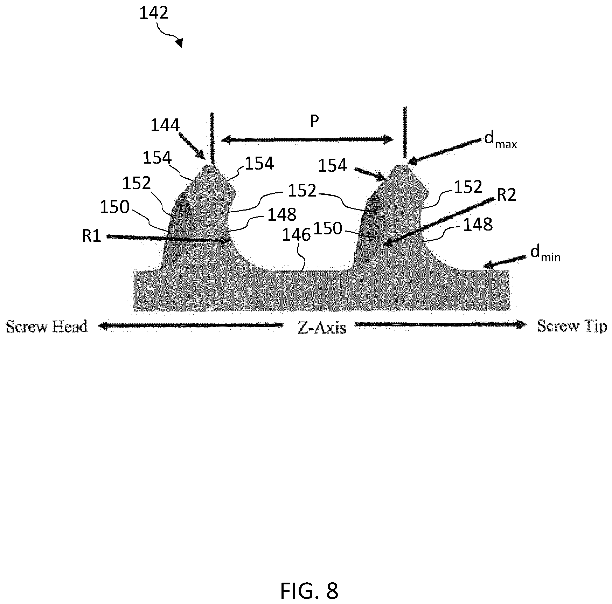

Turning now to FIGS. 7A-7B and 8, an orthopedic fixation device, implant, or bone fastener 140 is shown according to another embodiment. Similar to bone screws 10, 60, 100 and labeled with like elements, bone screw 140 includes enlarged head 16 and threaded shank 18 configured to engage bone. In this embodiment, radial undercuts 152 are provided on both sides of the thread 142 and the thread crest 144 is beveled or angled on both sides 154.

The shaft 18 of the screw 140 includes one or more threads 142. The screw thread 142 may include an external thread with a helical ridge defined by a helical groove wrapped around the shaft 18. Each screw thread section 142 has a thread crest 144, a root 146, and flanks 148, 150 that connect the crest 144 and adjacent roots 146. In this embodiment, both of the flanks 148, 150 may include undercuts 152. The undercut 152 on the thread form may act as a retention mechanism for bone fragments to help resist separation or improve pull-out strength. The undercuts 152 may include a concave surface including a curvature, bevel, or an area recessed inward and thereby defining the flanks 148, 150. The undercuts 152 may be generally circular, spherical, radial, or the like. In an exemplary embodiment best seen in FIG. 8, the undercuts 152 are radial undercuts 152 with a given root radius R1, R2. The first root radius R1 may be a front radius of the undercut 152 and the second root radius R2 may be a back root radius of the undercut 152. The front and back root radius R1, R2 may be the same or different. As shown, the root 146 may be generally flat or planar between the two radius R1, R2. It will be appreciated that the root 146 may be curved, sloped, or otherwise configured.

The thread crest 144 may optionally have one or more truncated, beveled, or angled surfaces 154. The tip of the crest 144 may have a generally planar or flat top. The sides of the crest 144 may include beveled or angled surfaces 154 having a given crest angle until intersecting the recessed undercuts 152. As shown, both sides 154 of the crest 144 may be sloped toward the Z-axis. It will be appreciated that the crest 144 and, if present, angled surfaces 154 may be rounded, curved, or otherwise configured.

The distal end 14 of the bone screw 140 may include a lead in or relief cut 156 to allow the undercut profile to preserve the bone upon entry of the fastener. Without a proper lead in, bone could be removed preventing the undercut 152 from engaging around the bone. The lead in 156 may include a truncated version of the thread 142 with a ramp-type geometry creating compression of bone before the hook portion begins. This lead in or relief cut 156 may help to preserve and strengthen the interface between the thread and bone. Although FIGS. 7A-7B depict a single lead embodiment, it will be appreciated that a similar lead in or relief cut may be applied to a multi-lead screw thread.

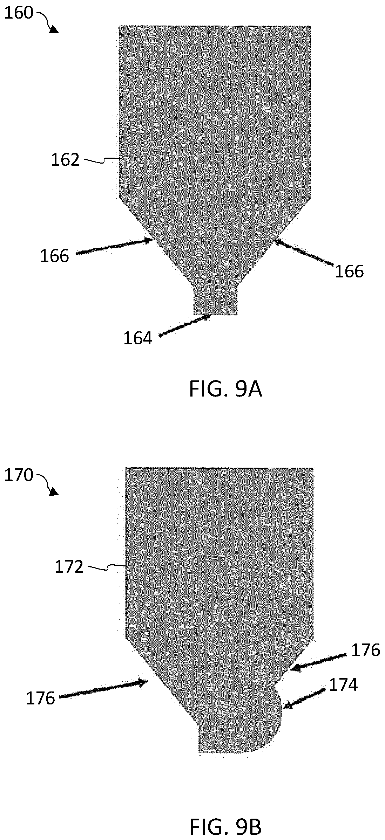

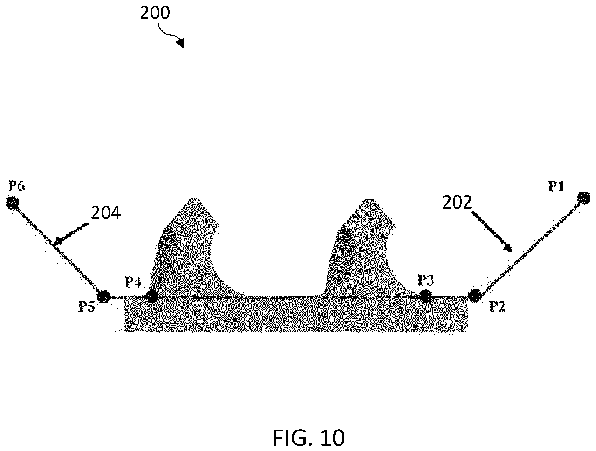

Turning now to FIGS. 9A-9D and 10, a manufacturing method including examples of cutting tools 160, 170, 180, 190 for forming the screw thread profiles are described. The thread 142 and undercut 152 may be formed with one or more industrial machining tools, such as lathe machines, grinding machines, drilling machines, shaping, and milling machines, used to cut down or finely shape different types of metals or materials. In an exemplary embodiment, the thread 142 may be formed using a lathe with one or more tool bits or custom single point cutting tools 160, 170, 180, 190. The custom tools 160, 170, 180, 190 may be staged in a sequence to cut the desired thread profile. As best seen in FIG. 10, these tools 160, 170, 180, 190 may follow a custom tool path to cut the desired thread.

FIG. 9A depicts a roughing tool 160 configured to start forming the thread profile. The roughing tool 160 may include a bit body 162 with one or more cutting surfaces 164, 166 at the distal end. The custom single point thread form of the roughing tool 160 may be configured to cut the thread minor dₘᵢₙ and crest angles 154, respectively. For example, the distal cutting surface 164 may be configured to cut the root 146 of the thread 142. The custom tool 160 may take several depths of cut in the X-axis or Y-axis until the desired thread minor dₘᵢₙ and thread depth D is achieved. When present, the angled side surfaces 166 may be configured to cut the crest angles 154 of the crest 144.

FIG. 9B shows a back radius form tool 170 configured to cut the back root radius R2 of the undercut 152. The back radius form tool 170 may include a bit body 172 with a curved cutting surface 174 and crest angle reliefs 176. The curved cutting surface 174 may be convexly shaped to cut the desired radius R2. The crest angle reliefs 176 may be angled to provide clearance between the cutting tool 170 and the previous relief cuts 154 of the workpiece. The custom single point form of the back root radius tool 170 may be used after the roughing tool 160 to take several depths of cut in the Z-axis until desired root radius R2 is achieved. This tool 170 may follow the custom tool path shown in FIG. 10.

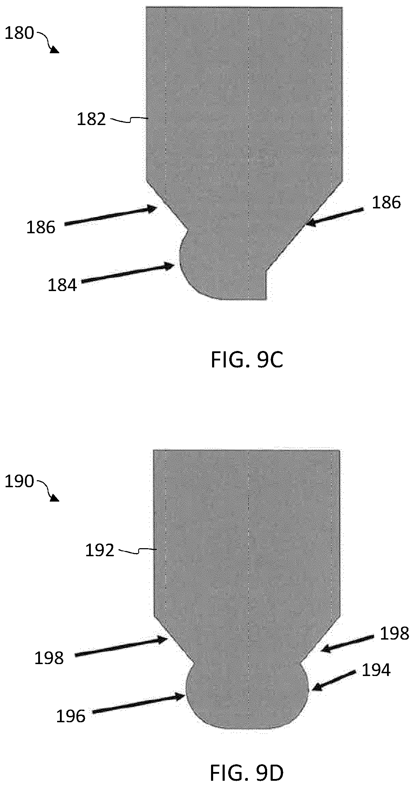

FIG. 9C shows a front radius form tool 180 configured to cut the front root radius R1 of the undercut 152. The front radius form tool 180 may include a bit body 182 with a curved cutting surface 184 and crest angle reliefs 186. The curved cutting surface 184 may be convexly shaped to cut the desired radius R1. The crest angle reliefs 186 may be angled to provide clearance between the cutting tool 180 and the previous relief cuts 154 of the workpiece. The custom single point form of the front root radius tool 180 may be used after the back radius form tool 170 to take several depth of cuts in the Z-axis until desired root radius R1 is achieved. This tool 180 may follow the custom tool path shown in FIG. 10.

FIG. 9D shows a dual radius form tool 190 where the front and back radius form tools 170, 180 are combined into a single custom form tool. Radius form tool 190 is configured to cut the front and back root radii R1, R2 simultaneously. The radius form tool 190 may include a bit body 192 with two opposed cutting surfaces 194, 196 and crest angle reliefs 198. The two curved cutting surfaces 194, 196 may be convexly shaped to cut the desired radii R1, R2. The crest angle reliefs 198 may be angled to provide clearance between the cutting tool 190 and the previous relief cuts 154 of the workpiece. The custom single point form of the dual root radius tool 190 may be used after the roughing tool 160 to take several depths of cut in the Z-axis until desired root radii R1, R2 is achieved. This tool 170 may follow the custom tool path shown in FIG. 10.

FIG. 10 shows one embodiment of a custom tool path 200 that may be used to create the desired thread profile. At the tool lead-in 200, the pitch/feed rate at point Pl is equal to the pitch as the desired thread pitch. The lathe feed rate may then adjust throughout the tool path 200 at the indicated locations P2 to P3, P4 to P5 before returning to the desired thread pitch/feed rate at point P6. The tool lead-out 204 is shown between locations P5 and P6. The adjustment of the feed rate throughout the tool path 200 ensures the root radius R1, R2 and radial undercut 152 is fully formed and does not remove any additional geometry. The number of points/feed rate adjustments can be added as needed to ensure the desired geometry is obtained. Although specific tools and tool path are shown in FIGS 9A-9D and 10 for creating thread 142, it will be appreciated that the tools and tool path may be modified to create other suitable thread profiles.

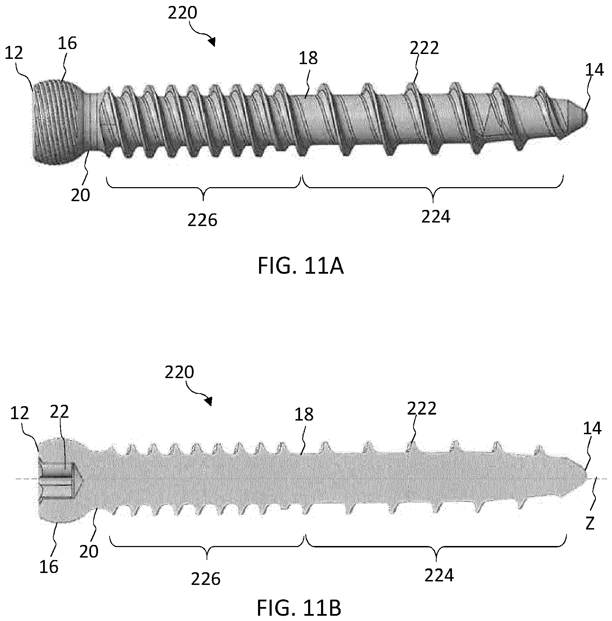

Turning now to FIGS. 11A-11B, an orthopedic fixation device, implant, or bone fastener 220 is shown according to another embodiment. Similar to bone screws 10, 60, 100, 140 and labeled with like elements, bone screw 220 extends from proximal end 12 to distal end 14 and includes enlarged head 16 and threaded shank 18 configured to engage bone. In this embodiment, the bone screw 220 may include a pedicle screw configured to optimize purchase in both cortical and cancellous bone. For example, the proximal portion of the screw 220 may be configured for cortical fixation and the distal portion of the screw 220 may be configured for cancellous fixation. The thread 222 may include a buttress thread that propagates from the distal tip 14 of the screw 220 as a one start or single lead 224 and then transitions into a two start or dual lead 226 in the middle section of the screw shaft 18. The distal thread 224 is configured to optimize screw purchase in cancellous bone while the proximal thread 226 is configured to optimize screw purchase in the denser cortical bone.

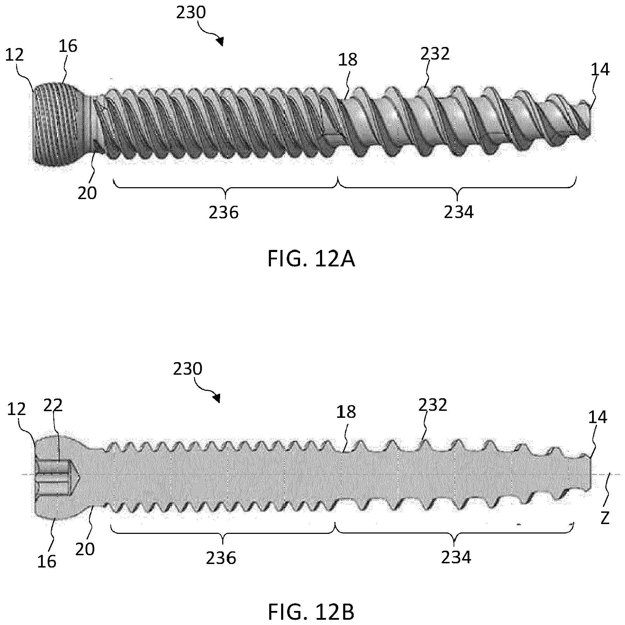

Turning now to FIGS. 12A-12B, an orthopedic fixation device, implant, or bone fastener 230 is shown according to another embodiment. In this embodiment, the thread 232 is a buttress thread that propagates from the distal tip 14 of the screw 230 as a two start or dual lead 234 and then transitions into a four start or quad lead 236 in the middle section of the screw shaft 18. The distal thread 234 is configured to optimize screw purchase in cancellous bone while the proximal thread 236 is configured to optimize screw purchase in the denser cortical bone.

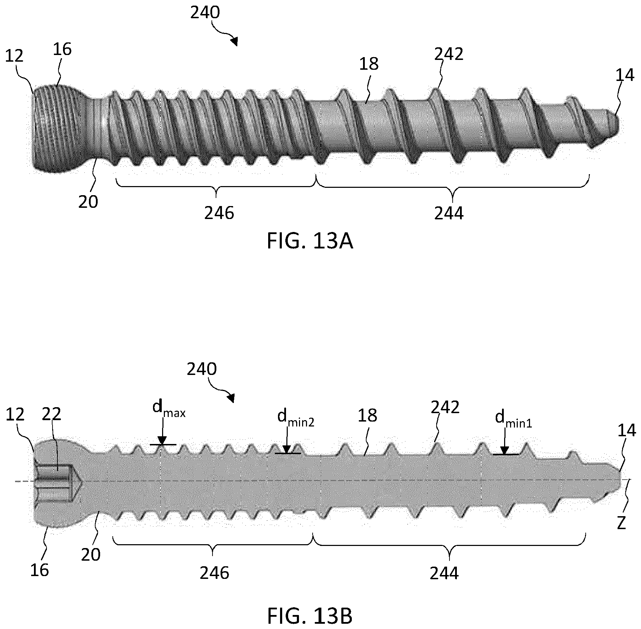

Turning now to FIGS. 13A-13B, an orthopedic fixation device, implant, or bone fastener 240 is shown according to another embodiment. Bone screw 240 includes a buttress thread that propagates from the distal tip 14 of the screw 240 as a one start or single lead 244 and then transitions into a two start or dual lead 246 in the middle section of the screw shaft 18. The distal thread 244 is configured to optimize screw purchase in cancellous bone while the proximal thread 246 is configured to optimize screw purchase in the denser cortical bone.

In addition to the varying thread lead and pitch, screw 240 includes multiple different core or minor diameters dₘᵢₙ₁, dₘᵢₙ₂. In other words, the distal single lead portion 244 may have a first minor diameter dₘᵢₙ₁ that is smaller than the second dual lead portion 246. For example, the single lead portion 244 may be dimensioned at about 2.0mm below the major diameter dₘₐₓ and the dual lead portion 246 may be dimensioned at about 1.5mm below the major diameter dₘₐₓ. This change in minor diameter dₘᵢₙ₁, dₘᵢₙ₂, coupled with pitch P, may help to optimize purchase in various bones densities where the deeper thread depth is reserved for the porous cancellous bone and the shallower thread depth is configured to optimize purchase in the dense cortical bone. A taper may be provided at the transition between the two minor diameters dₘᵢₙ₁, dₘᵢₙ₂ to facilitate a smooth insertion into bone. The size up at the minor diameter of the proximal threading may also help to provide a compressive effect of the screw 240 in the strong cortical bone, thereby resisting pullout and toggle.

The screw features and parameters may be modified or varied in bone screws 220, 230, 240 to meet the patient anatomy and desired clinical outcomes. In particular, the screw size, thread geometry, and relative dimensions may be varied. For example, the pedicle screw may be sized at a 6.5 mm listed diameter and 45 mm listed length. The sizes and dimensions may be scaled to appropriately fit screws varying in diameter from 4.0 mm up to 12.5 mm and lengths of 20 mm up to 140 mm.

Additionally, the screw tip geometry shown in bone screws 220, 230, 240 is one example. Other tip geometries may include a blunt tip, conical tip, pointed tip, awl tip, fluted tip, self-tapping tip, self-drilling tip, or the like. Bone screw 220, 230, 240 may further incorporate cannulated screw shanks and/or fenestrated screw shanks which allow for augmentation with bone cement or other suitable materials.

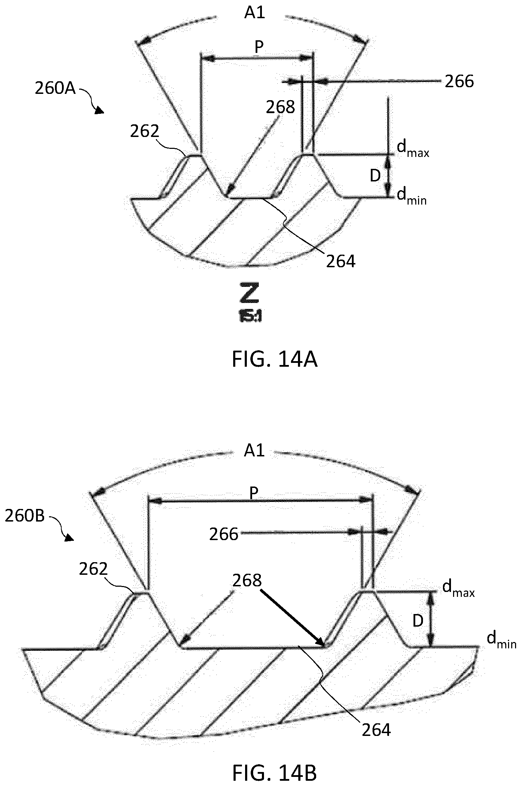

Turning now to FIGS. 14A-17B, various examples of thread forms or profiles are shown. The thread forms may range in angle, shape, and design. It will be appreciated that any of the thread forms described herein may be incorporated with any suitable bone fastener to achieve the intended function, such as enhanced screw purchase, improved pull-out strength, preventing lateral motions, improved bone lagging, reduced micromotion, reduced lateral separation, etc.

With further emphasis on FIGS. 14A-14B, the threads 260A, 260B may have a triangular thread form, thereby forming a generally V-thread with a crest 262 and a root 264. Thread 260B varies from thread 260A due to the thread pitch P and the thread depth D. The pitch P of thread 260B is greater than the pitch P of thread 260A. For example, the pitch P of thread 260B may be double the pitch P of thread 260A. The depth D of thread 260B may be greater than the depth D of thread 260A. The crest 262 may be truncated to have a crest flat 266 and the root 264 may also be generally flat (e.g., a generally cylindrical minor diameter dₘᵢₙ). The crest flat 266 may be the same for both threads 260A, 260B. The thread angle A1 is the included angle between adjacent thread flanks. As shown, the thread angle A1 may be about 60°. The thread angle A1 may be the same for both threads 260A, 260B. The transition between flank and root 264 may be radiused to form a rounded fillet surface 268.

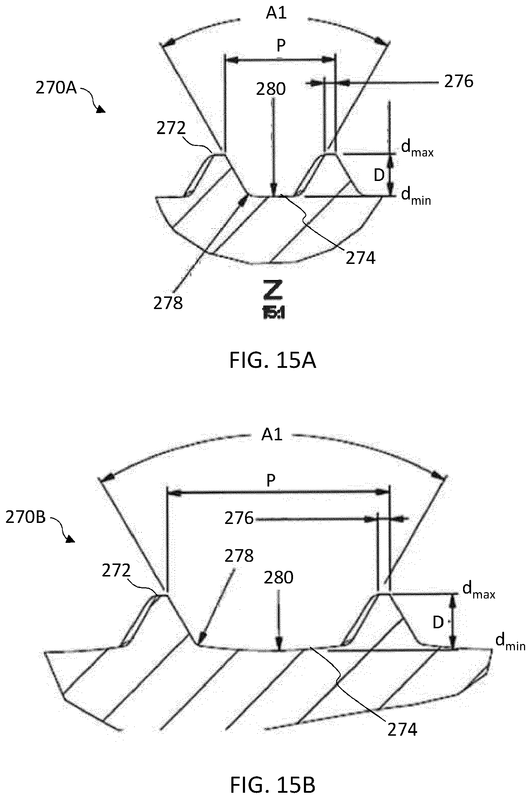

With further emphasis on FIGS. 15A-15B, the threads 270A, 270B may have a triangular thread form, thereby forming a generally V-thread with a crest 272 and a root 274. Thread 270B varies from thread 270A due to the thread pitch P and the thread depth D. The pitch P of thread 270B is greater than the pitch P of thread 270A. For example, the pitch P of thread 270B may be double the pitch P of thread 270A. The depth D of thread 270B may be greater than the depth D of thread 270A. In addition, threads 270A, 270B vary from threads 260A, 260B due to a curvature of root 274. In particular, the root diameter dₘᵢₙ follows a curve or radius 280. The root curve 280 may be the same for both threads 270A, 270B. The crest 272 may be truncated to have a crest flat 276. The length of crest flat 276 may be the same for both threads 270A, 270B. The thread angle A1 may be the same for both threads 270A, 270B. For example, the thread angle A1 may be about 60°. The transition between flank and root 274 may be radiused to form a rounded fillet surface 278. The degree of rounding for fillet surface 278 may be the same for both threads 270A, 270B.

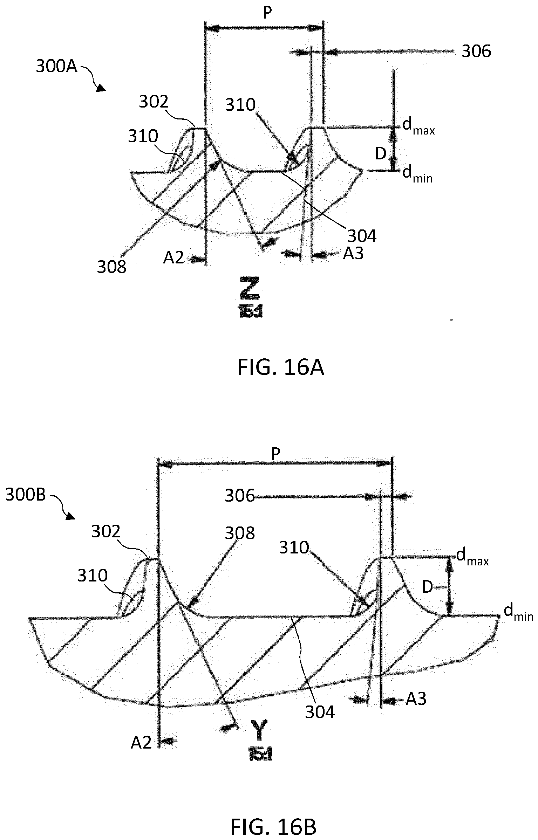

With further emphasis on FIGS. 16A-16B, the threads 300A, 300B may have a crest 302 and a root 304 with an undercut 310 in one or both of the flanks. Thread 300B varies from thread 300A due to the thread pitch P and the thread depth D. The pitch P of thread 300B is greater than the pitch P of thread 300A. For example, the pitch P of thread 300B may be double the pitch P of thread 300A. The depth D of thread 300B may be greater than the depth D of thread 300A. The flank angle A2 is the angle between a flank and a perpendicular thread axis. In this embodiment, the flank angle A2 may be about 25°. The flank angle A2 may be the same for both threads 300A, 300B. The helix angle A3 is the angle between the helix of the thread and a line parallel to the axis of rotation. In this embodiment, the helix angle A3 may be about 5°. The helix angle A3 may be the same for both threads 300A, 300B. The crest 302 may be truncated to have a crest flat 306. The length of crest flat 306 may be the same for both threads 300A, 300B. On one side, the transition between flank and root 304 may be radiused to form a rounded fillet surface 308. The degree of rounding for fillet surface 308 may be the same for both threads 300A, 300B. On the opposite side, the flank may include undercut 310. The undercut 310 may be a concave curvature such as a radial undercut in the base of the thread. The radius of curvature for undercut 310 may be the same for both threads 300A, 300B.

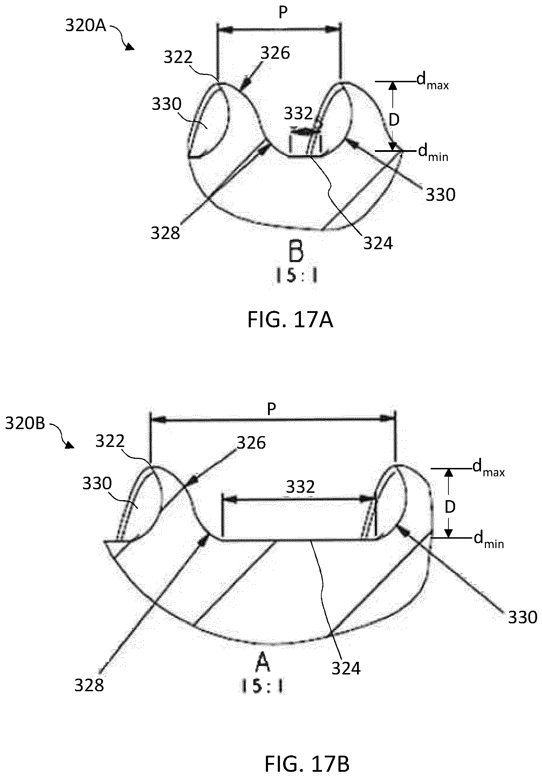

With further emphasis on FIGS. 17A-17B, the threads 320A, 320B may have a crest 322 and a root 324 with an undercut 330 in one or both of the flanks. Thread 320B varies from thread 320A due to the thread pitch P and the root flat length 332. The pitch P of thread 320B is greater than the pitch P of thread 320A. For example, the pitch P of thread 320B may be double the pitch P of thread 320A. The length of root flat 332 of thread 320B may be greater than the length of root flat 332 of thread 320A. For example, the root flat 332 may be about 0 in thread 320A and about 2.5 mm in thread 320B. The crest 322 may be rounded into the flank with a convex surface 326. The degree of curvature of convex surface 326 may be the same for both threads 320A, 320B. On the side with the convex surface 326, the transition between flank and root 324 may be radiused to form a rounded fillet surface 328. The degree of rounding for fillet surface 328 may be the same for both threads 320A, 320B. On the opposite side, the flank may include undercut 330. The undercut 330 may be a concave curvature such as a radial undercut removing the entire length of the original flank. The radius of curvature for undercut 330 may be the same for both threads 320A, 320B. This embodiment is similar to thread form 102 shown in FIG. 4. Threads 320A, 320B are configured to grab onto the bone and resist lateral forces, for example, due to the curved shape of the thread 320A, 320B.

In the case of lagging bone fragments together, the thread forms may act as a retention mechanism for the bone fragments to resist lateral separation. The bone fragments may resist pulling apart under normal loading conditions, which may more securely retain the bone reduction and provide a more stable environment for healing. The thread profile may keep the bone fragments lagged together for faster healing and may help to resist micromotion.

In the case of screws used with spinal constructs (e.g., attached to one or more spinal rods), the thread forms may help to relieve stresses within the bone, resist axial and lateral motions applied to the screw shank, and increase pull out strength. The spinal construct integrity may be maintained for better boney growth especially in osteoporotic bone. The screw thread may help to decrease motion of the screw within the bone to allow for faster boney re-growth and overall stronger spinal constructs.

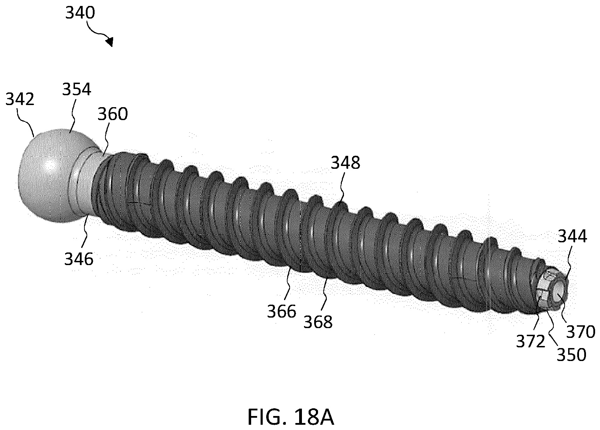

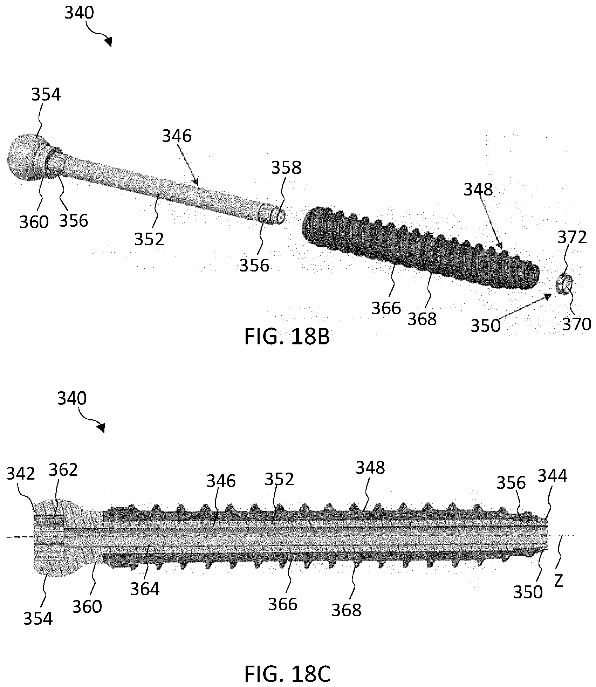

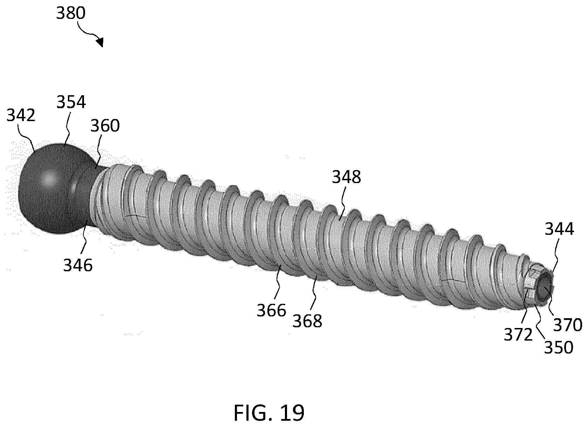

Turning now to FIGS. 18A-18C and 19, an orthopedic fixation device, implant, or bone fastener assembly 340, 380 is shown according to another embodiment. The implant 340, 380 extends from a proximal end 342 to a distal end 344 along a central longitudinal axis Z. The implant 340, 380 includes an inner core 346, an outer sleeve 348, and a cap 350 for securing the sleeve 348 to the core 346. The bone fastener assembly 340 may be configured to treat the following conditions: degenerative disc disease; spondylolisthesis; trauma (i.e., fracture or dislocation); and/or tumor. For example, the bone fastener assembly 340 may be a pedicle screw implant used in the thoracolumbar spine region for primary fixation.

In one embodiment, the implant 340, 380 is made partly or fully from carbon fiber or an equivalent robust radiolucent material. By utilizing one or more carbon fiber components, magnetic resonance imaging (MRI) and computerized tomography (CT) scans may be performed with minimal visual artifacts for better soft-tissue visualization. In addition, radiation therapy may be performed with minimal scattering of radiation beams, which may harm nearby healthy tissue. The multi-part assembly of implant 340, 380 may also offer superior fatigue strength, which benefits a patient population known for extended delays in healing. FIGS. 18A-18C depict an implant 340 with a titanium core 346 and a carbon fiber sleeve 348. The assembly 380 in FIG. 19 is identical to FIGS. 18A-18B, but the materials of the sleeve and core are swapped. Thus, implant 380 has a carbon fiber core 346 and a titanium sleeve 348. As such, the components of implant 340 will be described in further detail and it will be appreciated that the elements are the same for implant 380.

As best seen in the exploded view of FIG. 18B, implant 340 includes inner core 346, outer sleeve 348, and cap 350, which secures the sleeve 348 to the core 346. The core 346 includes an elongate rod 352 affixed to an enlarged head 354. The elongate rod 352 may be generally cylindrical or other suitable cross-section. One or more portions of the rod 352 may include engagement surfaces 356 configured to prevent rotation of the sleeve 348. The engagement surfaces 356 may include one or more planar surfaces, for example, forming a polygonal cross shape, such as a pentagon, hexagon, heptagon, octagon, etc. The engagement surfaces 356 may be provided at opposite ends of rod 352 to capture the proximal and distal ends of the sleeve 348. The distal end 356 of the rod 352 may be configured to secure cap 350. The enlarged head 354 may couple to the rod 352 via neck 360, for example, having a reduced diameter relative to the head 354 and a diameter greater than the rod 352. The head 354 and neck 360 may be similar to other screw geometries. For example, the head 354 may be smooth (as shown), threaded, or provided with a roughened or textured surface. The head 354 may be partially or fully rounded, cylindrical, spherical, or otherwise configured to engage bone and/or interface with a modular tulip element or head (e.g., for securing a spinal rod). The head 354 may define a driving recess 362, such as a hexalobular drive recess 22, configured to drive the implant 340 into bone. The core 346 may be cannulated with a central opening 364 extending therethrough between proximal and distal ends 342, 344 along central axis Z.

The sleeve 348 includes a body 366 with one or more threads 368 configured to engage bone. The body 366 may be generally cylindrical, tapered, or another suitable shape with a central cannulation configured to receive the rod 352 of the core 346. The body 366 defines one or more external threads 368 helically wound around the sleeve 348. The threads 368 may extend the entire length or a partial distance along the length of the sleeve 348. The threads 368 may include any of the threads described herein or any other suitable thread form.

During assembly, the sleeve 348 is inserted onto the core 346 from the distal end and is retained by the end cap 350. The end cap 350 may include a fastener, such as a nut, with a circular opening 370 configured to fit onto the distal end 358 of rod 352. The end cap 350 may be configured to permanently couple the sleeve 348 to the core 346. For example, the end cap 350 may be secured to the rod 352 with threads, crimps, adhesive, welding, or other suitable locking interface. The end cap 350 may vary in external geometry to aid in screw insertion. For example, the end cap 350 may include a taper, exterior threads, cutting flutes, de-corticating ribs, a sharp pointed tip, or other configurations. In the embodiment shown, the outer surface of the end cap 350 includes cutting flutes 372 configured to cut bone during insertion. All components are rotationally coupled, and the assembly 340 functions as a single unit, for example, as a pedicle screw.

The sleeve 348 and/or the core 346 may be comprised partly or fully from carbon fiber or an equivalent robust radiolucent material. In FIGS. 18A-18C, the bone screw 340 includes a titanium core 346, a titanium end cap 350, and a carbon fiber sleeve 348. In FIG. 19, the bone screw 380 includes a carbon fiber core 346, a titanium end cap 350, and a titanium sleeve 348. Alternatively, the screw 380 may be made from a single piece of carbon fiber and a thin shell of titanium or another implantable metal may surround the threads, any part of the screw, or the entire screw. This shell may be applied by any suitable method, such as spray coating.

The use of carbon fiber or other radiolucent material in the implant may help to improve post-operative MRI visibility, radiation therapy, and the overall fatigue life of the pedicle screw. In particular, the carbon fiber components may allow for MRI and CT scans to be performed with minimal visual artifacts for better soft-tissue visualization. In addition, radiation therapy may be performed with minimal scattering of radiation beams, which may harm nearby healthy tissue. The multi-part assembly of implant may also offer superior fatigue strength, which benefits a patient population known for extended delays in healing. Moreover, with the implant in multiple components, the assembly may have a modularity which may be helpful for manufacturing. For example, a single core that is compatible with a wide array of sleeves and end caps may allow for efficient manufacturing or a deep platform or different screw parts.

Although the invention has been described in detail and with reference to specific embodiments, it will be apparent to one skilled in the art that various changes and modifications can be made without departing from the spirit and scope of the invention. Thus, it is intended that the invention covers the modifications and variations of this invention provided they come within the scope of the appended claims and their equivalents. It is expressly intended, for example, that all components of the various devices disclosed above may be combined or modified in any suitable configuration.

The invention may be defined inter alia by the following aspects:
1. An orthopedic bone fastener comprising:
   a screw head and a shaft extending along a central longitudinal axis between a proximal end and a distal end, the screw head defining a drive recess and the shaft configured for engaging bone; and
   an external thread helically wound around the shaft, each thread section having a crest, a root, and leading and following flanks connecting the crest to adjacent roots, wherein the leading flank faces toward the distal end and is configured to enter into bone first and the following flank faces toward the proximal end of the bone fastener, and wherein at least one of the leading and following flanks comprises an undercut, thereby forming a hooked thread profile.
2. The fastener of aspect 1, wherein the undercut is a concave recessed surface with a radius of curvature extending between the crest and the root.
3. The fastener of aspect 1, wherein the undercut is a radial undercut.
4. The fastener of aspect 1, wherein the hooked thread profile extends along an entire length of the shaft.
5. The fastener of aspect 1, wherein the distal end of the bone fastener includes a relief cut configured to preserve bone upon entry of the bone fastener.
6. The fastener of aspect 1, wherein the crest is convexly curved.
7. The fastener of aspect 1, wherein the external thread includes a dual lead thread including a dual radial undercut.
8. An orthopedic bone fastener configured for lagging bone fragments together comprising:
   a screw head and a shaft extending along a central longitudinal axis between a proximal end and a distal end; and
   a dual lead thread helically wound around the shaft including a first thread section and a second thread section, each thread section having a crest, a root, and leading and following flanks connecting the crest to adjacent roots, wherein the leading flank faces toward the distal end and is configured to enter into bone first and the following flank faces toward the proximal end of the bone fastener, and wherein each of the first and second thread sections comprise an undercut, thereby forming a hook-like thread configured to help resist axial and lateral motions.
9. The fastener of aspect 8, wherein the dual lead thread has a repeating pattern of dual radial undercuts and V-shaped threads.
10. The fastener of aspect 8, wherein the first and second thread sections comprise dual radial undercuts facing toward one another.
11. The fastener of aspect 8, wherein an area between the first thread section and the second thread section forms a U-shaped groove such that the crests of adjacent thread sections point toward one another.
12. The fastener of aspect 8, wherein an area between the second thread section and the next first thread section forms a V-shaped groove such that the crests of adjacent thread sections point away from one another.
13. The fastener of aspect 8, wherein the first thread section includes an angled leading flank and a first undercut on the following flank.
14. The fastener of aspect 13, wherein the second thread section includes a second undercut on the leading flank and an angled following flank.
15. An orthopedic bone fastener configured to increase pullout strength comprising:
   a screw head and a shaft having a distal tip extending along a central longitudinal axis, the shaft having a proximal portion and a distal portion; and
   an external thread helically wound around the shaft, each thread section having a crest, a root, and leading and following flanks connecting the crest to adjacent roots, wherein the leading flank faces toward the distal tip and is configured to enter into bone first and the following flank faces toward the screw head of the bone fastener, and wherein the following flank comprises a hook configured to resist axial and lateral forces.
16. The fastener of aspect 15, wherein the external thread includes non-linear leading and following flanks.
17. The fastener of aspect 15, wherein the hook includes a circular recess.
18. The fastener of aspect 15, wherein the hook defines a radial undercut with a concave recessed surface extending between the crest and the root.
19. The fastener of aspect 15, wherein the leading flank comprises a convex curved profile.
20. The fastener of aspect 15, wherein the distal portion of the shaft includes a single lead thread configured to engage cancellous bone and the proximal portion of the shaft includes a double lead thread configured to engage cortical bone.

## Claims

1. An orthopedic bone fastener configured for lagging bone fragments together comprising:
a screw head and a shaft extending along a central longitudinal axis between a proximal end and a distal end; and
a dual lead thread helically wound around the shaft including a first thread section and a second thread section, each thread section having a crest, a root, and leading and following flanks connecting the crest to adjacent roots, wherein the leading flank faces toward the distal end and is configured to enter into bone first and the following flank faces toward the proximal end of the bone fastener, and wherein each of the first and second thread sections comprise an undercut, thereby forming a hook-like thread configured to help resist axial and lateral motions.

2. The fastener of claim 1, wherein the dual lead thread has a repeating pattern of dual radial undercuts and V-shaped threads.

3. The fastener of any one of the preceding claims, wherein the first and second thread sections comprise dual radial undercuts facing toward one another.

4. The fastener of any one of the preceding claims, wherein an area between the first thread section and the second thread section forms a U-shaped groove such that the crests of adjacent thread sections point toward one another.

5. The fastener of any one of the preceding claims, wherein an area between the second thread section and the next first thread section forms a V-shaped groove such that the crests of adjacent thread sections point away from one another.

6. The fastener of any one of the preceding claims, wherein the first thread section includes an angled leading flank and a first undercut on the following flank.

7. The fastener of claim 6, wherein the second thread section includes a second undercut on the leading flank and an angled following flank.

8. An orthopedic bone fastener configured to increase pullout strength comprising:
a screw head and a shaft having a distal tip extending along a central longitudinal axis, the shaft having a proximal portion and a distal portion; and
an external thread helically wound around the shaft, each thread section having a crest, a root, and leading and following flanks connecting the crest to adjacent roots, wherein the leading flank faces toward the distal tip and is configured to enter into bone first and the following flank faces toward the screw head of the bone fastener, and wherein the following flank comprises a hook configured to resist axial and lateral forces.

9. The fastener of claim 8, wherein the external thread includes non-linear leading and following flanks.

10. The fastener of claim 8 or claim 9, wherein the hook includes a circular recess.

11. The fastener of any one of claims 8 to 10, wherein the hook defines a radial undercut with a concave recessed surface extending between the crest and the root.

12. The fastener of any one of claims 8 to 11, wherein the leading flank comprises a convex curved profile.

13. The fastener of any one of claims 8 to 12, wherein the distal portion of the shaft includes a single lead thread configured to engage cancellous bone and the proximal portion of the shaft includes a double lead thread configured to engage cortical bone.
